# EUROPÄISCHE PATENTSCHRIFT

(11) **EP 1 106 997 B1**
(45) Veröffentlichungstag und Bekanntmachung des Hinweises auf die Patenterteilung: **14.03.2012**
(21) Anmeldenummer: 00811055.3
(22) Anmeldetag: 09.11.2000
(51) Int. Cl.: G01N 27/02, G01N 27/22, G01N 33/18

(54) **Verfahren zur Oel-in-Wasser Messung**
Method for measuring oil in water
Procédé pour la mesure d'huile dans l'eau

(30) Priorität: 08.12.1999 DE 19959005
(43) Veröffentlichungstag der Anmeldung: 13.06.2001
(73) Patentinhaber: Carnelian Cordless LLC, Las Vegas, NV 89119 (US)
(72) Erfinder: Matter, Daniel, 5200 Brugg (CH); Rüegg, Walter, 5304 Endingen (CH); Kleiner, Thomas, 5442 Fislisbach (CH); Byatt, John Anthony, 5313 Klingnau (CH)
(74) Vertreter: Small, Gary James

(56) Entgegenhaltungen:
- DE-A- 3 617 598
- DE-C- 3 542 238
- GB-A- 2 322 937
- US-A- 4 034 219
- US-A- 4 057 721

## Beschreibung

### TECHNISCHES GEBIET

Die vorliegende Erfindung bezieht sich auf das Gebiet der Messung kleiner Ölkonzentrationen in Wasser. Sie geht aus von einem Verfahren zur Öl-in-Wasser Messung nach dem Oberbegriff der Anspruch 1.

### STAND DER TECHNIK

Für die Offshore-Erdölförderung werden seit kurzem Hochdruck-Separatortanks entwickelt, die zur Trennung der Phasen Sand, Wasser, Öl und Gas umittelbar am Meeresgrund geeignet sind. Das ins Meer zurückgepumpte Wasser darf nach gesetzlichen Bestimmungen eine Ölkonzentration von maximal wenigen 10 ppm aufweisen. Mit herkömmlichen kapazitiven oder optischen Öl-in-Wasser Sensoren kann zwar prinzipiell eine solche hohe Messempfindlichkeit, jedoch nicht die erforderliche Wartungsfreiheit, Langlebigkeit sowie Druck-und Temperaturfestigkeit erreicht werden.

Eine derartige Vorrichtung zur Bestimmung einer geringen Ölkonzentration in Wasser ist aus dem U. S. Pat. No. 4,137,494 bekannt. Die Messzelle besteht aus einer ölabsorbierenden Membran mit beidseitig aufgebrachten Elektroden. Im Wasser enthaltene Ölmoleküle dringen in die Membran ein, verdrängen dort eingelagerte Ionen und erhöhen den elektrischen Widerstand der Membran. Die Widerstandsänderung ist dann ein Mass für die Ölkonzentration im durchströmenden Wasser. Zur Steigerung der Messempfindlichkeit muss eine Wasserprobe in einem geschlossenen Kreislauf mehrfach durch die Membran gepumpt werden, um dort sämtliches Öl zu absorbieren. Das Verfahren hat wesentliche Nachteile. Sobald die total akkumulierte Ölmenge einen Sättigungswert erreicht, muss die Membran entsorgt oder zur Regeneration mit einem Lösungsmittel gespült werden. Daher sind periodische Wartungsintervalle zwingend erforderlich, wodurch ein Einbau an unzugänglichen Stellen, z. B. auf dem Meeresgrund, erschwert oder verunmöglicht wird. Auch kann die Messung durch eine veränderliche Ionenkonzentration im Wasser verfälscht werden.

Ein Verfahren gemäß dem Oberbegriff des Anspruchs 1 ist durch DE 35 42 238 bereits bekannt.

### DARSTELLUNG DER ERFINDUNG

Aufgabe der vorliegenden Erfindung ist es, ein Verfahren zur Öl-in-Wasser Messung anzugeben, bei welchem eine erhöhte Messgenauigkeit im Vergleich zu DE 35 42 238 realisierbar ist. Diese Aufgabe wird erfindungsgemäss durch die Merkmale des Anspruchs 1 gelöst.

Durch das Trennfilter kann auf einfachste Weise sowohl die Messempfindlichkeit erhöht als auch die Kalibrationsflüssigkeit bereit gestellt werden.

Die erfindungsgemässe Lösung besteht auch in einem Verfahren zur Öl-in-Wasser Messung mit einer Messzelle, deren Impedanz ein Mass für eine Ölkonzentration im durchströmenden Wasser oder in einem Akkumulationsfilter ist, wobei die Messzelle in wiederkehrenden Zeitabständen automatisch kalibriert und/oder gespült wird. Bei der Kalibration wird mit einer bekannten Referenzflüssigkeit ein aktueller Referenz-Impedanzwert der Messzelle gemessen. Bei der Spülung wird die Messzelle selbsttätig von Ölrückständen gereinigt. Sowohl Selbstkalibration als auch Selbstspülung werden von einer geeignet ausgelegten oder programmierten Steuerelektronik ausgeführt. Vorteile sind u. a. eine Verringerung der Signaldrift, eine verbesserte Langzeitzuverlässigkeit und eine lange Betriebsdauer ohne Überwachung oder Filteraustausch. Insgesamt wird erstmals ein Öl-in-Wasser Sensor angegeben, der für einen Einsatz an schwer zugänglichen Orten bei der Erdölförderung o. ä. geeignet ist.

Gemäss einem Ausführungsbeispiel wird die Messzelle alternierend in einem langen Messzyklus (z. B. 10 Minuten) und einem kurzen Spülzyklus (z. B. 1 Minute) und/oder einem noch kürzeren Kalibrierzyklus betrieben. Die Messzelle weist zwei Akkumulationsfilter auf, deren Messzyklen einander so ergänzen, dass weitgehend zu jedem Zeitpunkt ein Messsignal in der Messzelle bestimmbar ist. Auf diese Weise wird eine grosse Verfügbarkeit der Messzelle für on-line Messungen erzielt.

In einem anderen Ausführungsbeispiel ist in einer kapazitiven Messzelle ein Ölakkumulationsfilter angeordnet, welches durch ein ventilgesteuertes Leitungssystem zum Spülen in Rückwärtsrichtung durchströmbar ist. Durch die Richtungsumkehr beim Spülvorgang können Ölreste oder ein Filterkuchen auf der Filteraussenfläche, d. h. auf der Einlassseite beim Messzyklus, auch mit ölkontaminiertem Wasser sehr effizient herausgespült werden.

Das Ölakkumulationsfilter kan ein zylindrisches Ringfilter aus poröser Keramik oder Polyäthylenfibrid sein, welches mindestens je einen radialen und axialen Anschluss aufweist. Durch die Zylinderform wird ein mechanisch robustes, kompaktes und zugleich grossflächiges Akkumulationsfilter geschaffen. Zusätzlich können durch Segmentierung der Elektroden auf der äusseren und inneren Zylindermantelfläche mehrere Filter auf einem gemeinsamen Keramikkörper realisiert sein, die einander zeitlich ergänzende Messzyklen, intermittierende Spülzyklen und/oder Zyklen zur Offsetbestimmung durchlaufen. Dadurch können die Selbstspülung und Selbstkalibration in einem einzigen Bauteil realisiert werden.

Weitere Ausführungen, Vorteile und Anwendungen der Erfindung ergeben sich aus den abhängigen Ansprüchen sowie aus der nun folgenden Beschreibung anhand der Figuren.

### KURZE BESCHREIBUNG DER ZEICHNUNGEN

Es zeigen:
- Fig. 1: einen Öl-in-Wasser Sensor mit einem Querströmungsfilter zur Ölanreicherung für eine kapazitive Durchfluss-Messzelle;
- Fig. 2: einen Öl-in-Wasser Sensor, der eine kapazitive Messzelle mit ölakkumulierendem Keramikfilter aufweist;
- Fig. 3: eine serielle Anordnung zweier Ölakkumulationsfilter zum simultanen Messen und Spülen und zur unabhängigen Offsetmessung; und
- Fig. 4: eine kapazitive Messzelle mit einem Ringzylinderfilter.

In den Figuren sind gleiche Teile mit gleichen Bezugszeichen versehen.

### WEGE ZUR AUSFÜHRUNG DER ERFINDUNG

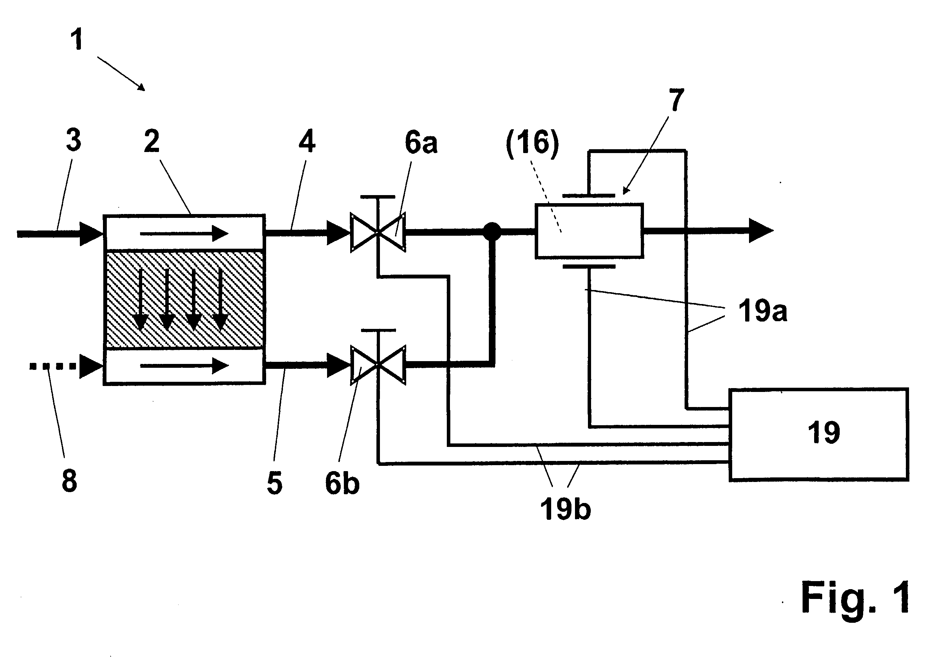

Die Erfindung hat ein Verfahren zur Öl-in-Wasser Messung zum Gegenstand, das besonders zur Messung einer Ölkonzentration in Wasser in einem Hochdruck-Separatortank geeignet ist. Einer Messzelle 7, 12 wird ölkontaminiertes Wasser zugeführt und ein elektrisches Impedanzsignal der Messzelle 7, 12 gemessen. Erfindungsgemäss wird die Messzelle 7, 12 in periodischen Intervallen automatisch kalibriert und/oder gespült. Im folgenden werden Ausführungsbeispiele zum Verfahren unter genereller Bezugnahme auf die Fig. 1-3 angegeben.

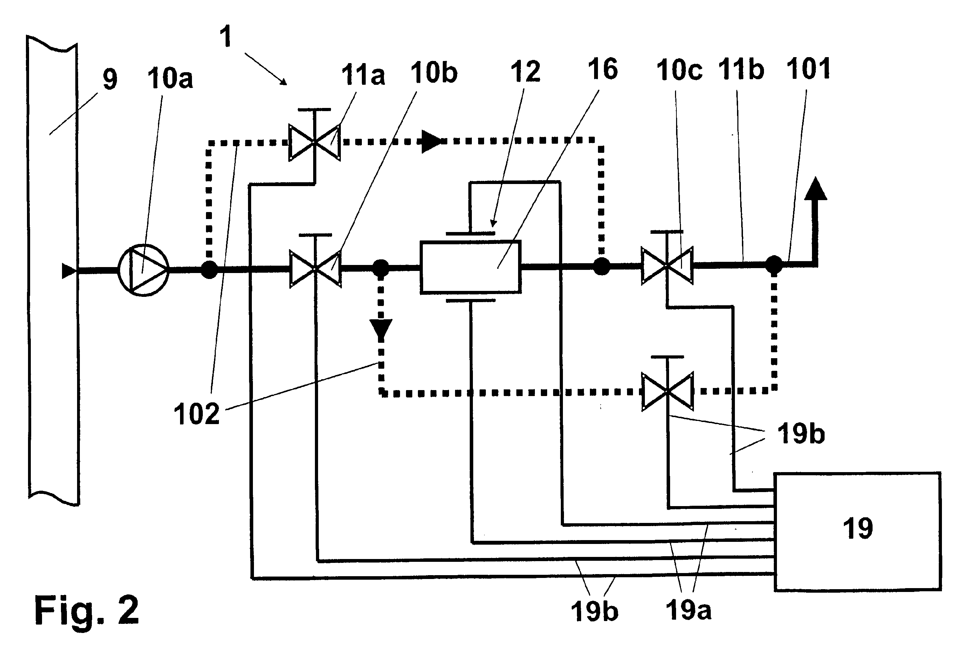

Beim Kalibrieren wird die Messzelle 7, 12 mit sauberem Wasser durchströmt und ein Kalibriersignal gebildet (Fig. 1). Beim Spülen wird die Messzelle 12 rückwärts mit Wasser durchströmt (Fig. 2). Erfindungsgemäss kann die Rückwärtsspülung sogar mit ölkontaminiertem Wasser durchgeführt werden. Dadurch wird der Öl-in-Wasser Sensor z. B. für Anwendungen in der Erdölindustrie bedeutend vereinfacht und die Alltagstauglichkeit verbessert. Es kann jedoch auch ein zur Spülung geeignetes Lösungsmittel aus einem nicht dargestellten Zusatztank zugeführt werden. Alternativ oder ergänzend kann das Akkumulationsfilter 16, 16a, 16b der Messzelle 12 durch Ultraschall gereinigt werden.

Vorzugsweise wird in der Messzelle 7, 12 ein Kapazitätssignal als Mass für die Ölkonzentration im Wasser verwendet. In einer Durchfluss-Messzelle 7 wird eine Ölkonzentration des durchströmenden Wassers gemessen. In einer Akkumulations-Messzelle 12 wird in einem Akkumulationsfilter 16, 16a, 16b eine akkumulierte Ölkonzentration gemessen. In beiden Fällen kann in einem vorgeschalteten Trennfilter 2 eine Ölkonzentration im Wasser um einen bekannten oder bestimmbaren Faktor angereichert werden.

Die Funktionsweise des Sensors 1 beruht darauf, dass durch den Ölanteil im Wasser die Kapazität gegenüber sauberem Wasser erniedrigt wird. Mit Akkumulationsfilter 16, 16a, 16b gibt ein absolutes Signal die gesamte eingelagerte Ölkonzentration und ein differentielles Signal die momentan einströmende Ölkonzentration an.

Ein Intervall soll einen längeren Messzyklus und einen kürzeren Kalibrier- und/oder Spülzyklus umfassen. Die Messzelle 7, 12 kann auch mehrere Akkumulationsfilter 16a, 16b mit alternierenden Mess- und Spülzyklen umfassen, wobei die Messzyklen einander derart zeitlich ergänzen oder überlappen, dass permanent ein Impedanzsignal der Messzelle 7, 12 messbar ist, und die Spülzyklen einander derart abwechseln, dass jedes Filter 16a, 16b hinreichend lange gespült wird.

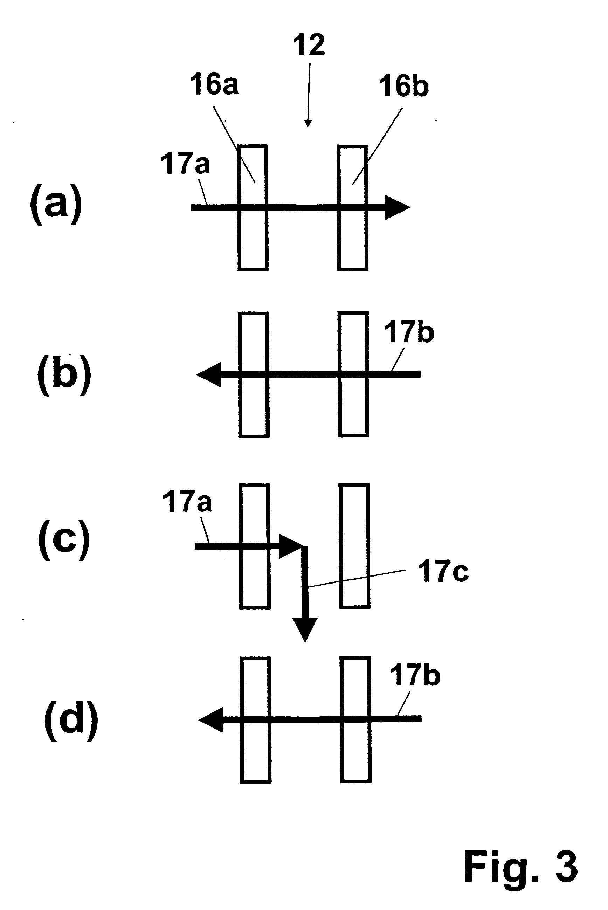

Im Ausführungsbeispiel nach Fig. 3 besitzt die Messzelle 7, 12 genau zwei Akkumulationsfilter 16a, 16b, die in Serie hintereinander durchströmt werden. In einem ersten Arbeitsgang (Fig. 3a) durchläuft das erste Akkumulationsfilter 16a einen Messzyklus und das zweite Akkumulationsfilter 16b einen Kalibrier- und/oder Spülzyklus. In einem zweiten Arbeitsgang (Fig. 3b) durchläuft das erste Akkumulationsfilter 16a einen Kalibrier- und/oder Spülzyklus und das zweite Akkumulationsfilter 16b einen Messzyklus. In einem weiteren Arbeitsgang (Fig. 3c) kann zur Feststellung eines Offsetsignals nur ein Akkumulationsfilter 16a durchströmt und am anderen Akkumulationsfilter 16b eine Offsetmessung durchgeführt werden. Danach wird wieder ein zweiter Arbeitsgang (Fig. 3d), nun jedoch mit neuem Offset am Filter 16b, ausgeführt.

In dieser Konfiguration wird also in der Messzelle 7, 12 permanent ein aktuelles Messsignal erzeugt und zugleich das nicht messende Akkumulationsfilter 16a oder 16b gespült und/oder als Mass für die total eingelagerte Ölmenge sein neuer Offsetwert bestimmt. Dadurch wird eine hohe Verfügbarkeit und lange Betriebsdauer ohne manuelle Wartung gewährleistet.

Gemäss Fig. 1 und 2 umfasst der Sensor 1 Mittel 4, 6a; 10a-10c, 101 zur Zuführung von ölkontaminiertem Wasser zu einer Messzelle 7, 12, die mit Messmitteln 19, 19a zur Impedanzmessung verbunden ist. Der Sensor 1 umfasst ferner . Mittel 2, 5, 6b, 8; 10b, 10c, 11a, 11b, 102 zur Selbstkalibration und/oder zur Selbstspülung der Messzelle 7, 12 und Steuermittel 19, 19b zu deren automatischer Aktivierung. Es folgen detailiertere Ausführungsbeispiele.

Gemäss Fig. 1 können die Mittel zur Selbstkalibration 2, 5, 6b, 8 ein Querströmungsfilter 2 mit einem Einlass 3 für ölkontaminiertes Wasser umfassen. Der erste Auslass 4 für ölangereichertes Wasser und der zweite Auslass 5 für gereinigtes Wasser sind über steuerbare Ventile 6a, 6b alternierend mit der Messzelle 7, 12 verbindbar. Mit Vorteil weist das Querströmungsfilter 2 zur seiner eigenen Reinigung einen zweiten Einlass 8 für sauberes Wasser auf.

Gemäss Fig. 2 können die Mittel zur Selbsspülung 10b, 10c, 11a, 11b, 102 zwei zur Messzelle 12 parallele Spülleitungen 102 umfassen, wobei die Messzelle 12 durch erste Ventile 10b, 10c von einer Messleitung 101 trennbar und über zweite Ventile 11a, 11b mit den Spülleitungen 102 in Rückspülrichtung verbindbar ist. 9 bezeichnet eine Zuleitung von einem Separatortank und 10a eine Pumpe. Mit Vorteil umfassen die Steuermittel 19, 19b eine Steuerelektronik 19 und Steuersignalleitungen 19b, die zur automatischen Steuerung der Ventile 10b, 10c; 11a, 11b ausgestaltet sind.

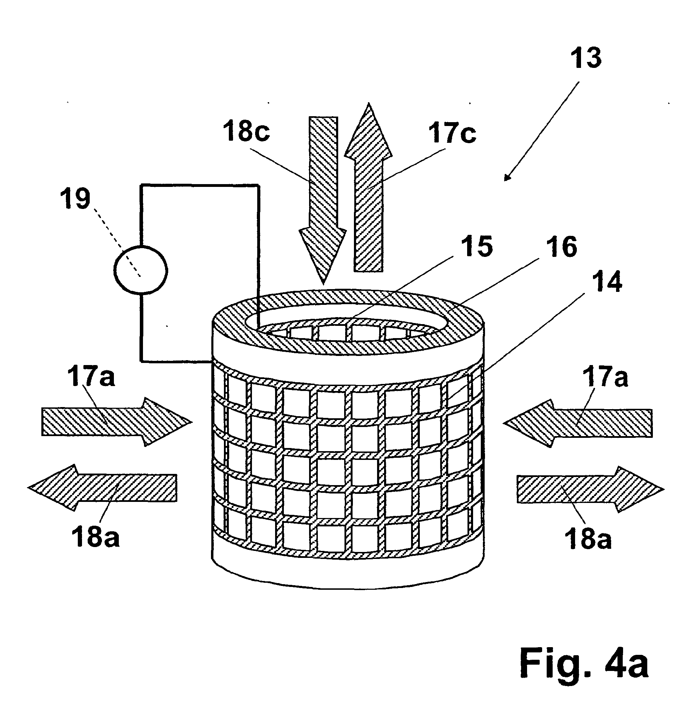

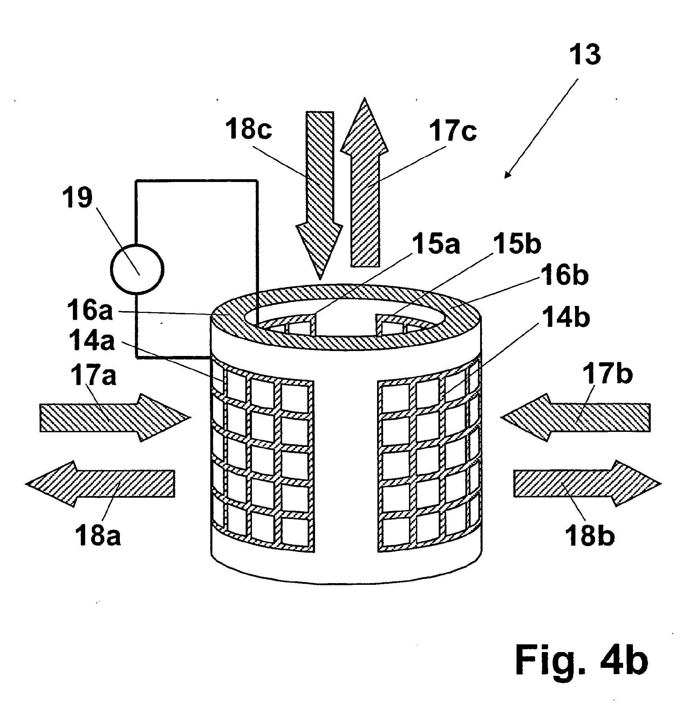

Gemäss Fig. 4 ist ein Akkumulationsfilter 16 der Messzelle 7, 12 ein zylindrisches Ringfilter 13, das eine Aussenelektrode 14 und eine Innenelektrode 15 auf dem Zylindermantel sowie einen radialen Anschluss 17a, 18a und einen axialen Anschluss 17c, 18c aufweist (Fig. 4a). Das erfindungsgemässe Zylinderfilter 13 zeichnet sich durch eine grosse Filterfläche, Druckfestigkeit, kompakte Bauweise und einfache Einbaubarkeit aus.

Mit Vorteil weist das zylindrische Ringfilter 13 je zwei halbschalenförmige Aussenelektroden 14a, 14b und Innenelektroden 15a, 15b sowie zwei getrennte radiale Anschlüsse 17a, 18a; 17b, 18b auf (Fig. 4b). Darüberhinaus können auch auf einfache Weise mehrere radiale Anschlüsse für mehrere Teilfilter 16a, 16b mit entsprechend segmentierten Elektroden 14a, 14b, 15a, 15b realisiert sein. Ferner sind zwei axiale Anschlüsse nach oben und unten machbar.

Das Akkumulationsfilter 16, 16a, 16b der Messzelle 7, 12 soll ein Keramikfilter 16, 16a, 16b mit wasserdurchlässiger, ölabsorbierender Porengrösse oder ein Polyäthylenfibridfilter sein. Die poröse Keramik dient vorwiegend zur mechanischen Abscheidung von Ölen oder allgemein Kohlenwasserstoffen oder (toxischen) Additiven. Im Polyäthylenfibridfilter werden Kohlenwasserstoffe auch durch ihre chemische Affinität zum Filtermaterial eingelagert. Das Trennfilter 2 kann entsprechend aufgebaut sein.

Durch die Erfindung wird ein alltagstauglicher, langlebiger Öl-in-Wasser Sensor 1 angegeben, der zu kontinuierlichen Echtzeitmessungen der Ölkontamination im 10 ppm Bereich geeignet und an unzugänglichen Orten einsetzbar ist.

### BEZUGSZEICHENLISTE

- 1: Öl-in-Wasser Sensor
- 2: Trennfilter, Querströmungsfilter
- 3: Einlass
- 4: erster Auslass
- 5: zweiter Auslass
- 6a, 6b: Ventile
- 7, 12: kapazitive Messzelle
- 8: zweiter Einlass
- 9: Zuleitung von Separatortank
- 101: Durchflussleitung, Messleitung
- 102: Rückflussleitung, Spülleitung
- 10a: Pumpe
- 10b, 10c: Ventile
- 11a, 11b: Ventile
- 13: kapazitives Ringfilter, Keramikfilter
- 14: Aussenelektrode
- 15: Innenelektrode
- 16: Keramikfilter, Kunststofffilter
- 16a, 16b: Akkumulationsfilter, Ölakkumulator
- 17a, 17b: radiale Einströmung
- 18a, 18b: radiale Ausströmung
- 17c: axiale Ausströmung
- 18c: axiale Einströmung
- 19: Messapparatur, Mess- und Steuerelektronik
- 19a: Messsignalleitungen
- 19b: Steuersignalleitungen

## Patentansprüche

1. Verfahren zur Öl-in-Wasser Messung, , wobei ölkontaminiertes Wasser einer Messzelle (7, 12) zugeführt wird und ein elektrisches Impedanzsignal der Messzelle (7, 12) gemessen wird, und wobei die Messzelle (7, 12) in periodischen Intervallen automatisch kalibriert und/oder gespült wird, **dadurch gekennzeichnet, dass**
a) die Messzelle (7, 12) genau zwei Akkumulationsfilter (16a, 16b) aufweist, die in Serie hintereinander durchströmt werden,
b) in einem ersten Arbeitsgang das erste Akkumulationsfilter (16a) in einem Messzyklus und das zweite Akkumulationsfilter (16b) in einem Kalibrier-und/oder Spülzyklus betrieben wird,
c) in einem zweiten Arbeitsgang das erste Akkumulationsfilter (16a) in einem Kalibrier- und/oder Spülzyklus und das zweite Akkumulationsfilter (16b) in einem Messzyklus betrieben wird und
d) insbesondere dass zur Feststellung eines Offsetsignals nur ein Akkumulationsfilter (16a) durchströmt wird und am anderen Akkumulationsfilter (16b) eine Offsetmessung durchgeführt wird.

2. Verfahren nach Anspruch 1, **dadurch gekennzeichnet, dass**
a) ein Intervall einen längeren Messzyklus und einen kürzeren Kalibrier- und/oder Spülzyklus umfasst und/oder
b) die Messzelle (7, 12) mehrere Akkumulationsfilter (16a, 16b) mit alternierenden Mess- und Spülzyklen umfasst, die einander derart ergänzen, dass permanent ein Impedanzsignal der Messzelle (7, 12) gemessen wird.

3. Verfahren nach einem der Ansprüche 1-2, **dadurch gekennzeichnet, dass**
a) beim Kalibrieren die Messzelle (7, 12) mit sauberem Wasser durchströmt und ein Kalibriersignal gebildet wird und/oder
b) beim Spülen die Messzelle (12) rückwärts mit Wasser, insbesondere mit ölkontaminiertem Wasser, oder mit einem Lösungsmittel durchströmt wird und/oder
c) beim Spülen ein Akkumulationsfilter (16, 16a, 16b) der Messzelle (12) durch Ultraschall gereinigt wird.

4. Verfahren nach einem der Ansprüche 1-3, **dadurch gekennzeichnet, dass**
a) in der Messzelle (7, 12) ein Kapazitätssignal als Mass für die Ölkonzentration im Wasser verwendet wird und/oder
b) in der Messzelle (7) eine Ölkonzentration des durchströmenden Wassers oder eine in einem Akkumulationsfilter (16, 16a, 16b) der Messzelle (7, 12) akkumulierte Ölkonzentration gemessen wird und/oder
c) in einem vorgeschalteten Trennfilter (2) eine Ölkonzentration im Wasser um einen bekannten Faktor angereichert wird.

## Claims

1. Method for measuring oil in water, wherein water contaminated with oil is fed to a measuring cell (7, 12) and an electrical impedance signal of the measuring cell (7, 12) is measured, and wherein the measuring cell (7, 12) is automatically calibrated and/or rinsed in periodic intervals, **characterized in that**
a) the measuring cell (7, 12) has precisely two accumulation filters (16a, 16b) through which a flow passes in series,
b) in a first working step the first accumulation filter (16a) is operated in a measuring cycle and the second accumulation filter (16b) is operated in a calibration cycle and/or rinsing cycle,
c) in a second working step the first accumulation filter (16a) is operated in a calibration cycle and/or rinsing cycle and the second accumulation filter (16b) is operated in a measuring cycle, and
d) in particular **in that**, in order to detect an offset signal, there is a flow through just one accumulation filter (16a), and an offset measurement is carried out at the other accumulation filter (16b).

2. Method according to Claim 1, **characterized in that**
a) an interval comprises a relatively long measuring cycle and a relatively short calibration cycle and/or rinsing cycle
and/or
b) the measuring cell (7, 12) comprises a plurality of accumulation filters (16a, 16b) with alternating measuring and rinsing cycles which supplement one another in such a way that an impedance signal of the measuring cell (7, 12) is continuously measured.

3. Method according to one of Claims 1-2, **characterized in that**
a) during the calibration process clean water flows through the measuring cell (7, 12) and a calibration signal is formed, and/or
b) during the rinsing process water, in particular water contaminated with oil or with a solvent, flows backwards through the measuring cell (12), and/or
c) during the rinsing process an accumulation filter (16, 16a, 16b) of the measuring cell (12) is cleaned by means of ultrasound.

4. Method according to one of Claims 1-3, **characterized in that**
a) a capacitance signal is used in the measuring cell (7, 12) as a measure for the concentration of oil in the water, and/or
b) a concentration of oil in the water flowing through or a concentration of oil which has accumulated in an accumulation filter (16, 16a, 16b) of the measuring cell (7, 12) is measured in the measuring cell (7), and/or
c) a concentration of oil in the water is enriched by a known factor in a separating filter (2) which is arranged upstream.

## Revendications

1. Procédé de mesure d'huile dans l'eau, dans lequel de l'eau contaminée par de l'huile est apportée à une cellule de mesure (7, 12) et le signal d'impédance électrique de la cellule de mesure (7, 12) est mesuré, la cellule de mesure (7, 12) étant étalonnée et/ou rincée automatiquement à des intervalles périodiques, **caractérisé**
a) **en ce que** la cellule de mesure (7, 12) présente exactement deux filtres à accumulation (16a, 16b) traversés l'un après l'autre en série,
b) **en ce que** dans une première étape de travail, le premier filtre à accumulation (16a) est conduit dans un cycle de mesure et le deuxième filtre à accumulation (16b) dans un cycle d'étalonnage et/ou de rinçage,
c) **en ce que** dans une deuxième étape de travail, le premier filtre à accumulation (16a) est conduit dans un cycle d'étalonnage et/ou de rinçage et le deuxième filtre à accumulation (16b) est conduit dans un cycle de mesure et
d) **en ce qu'**en particulier pour déterminer un signal de décalage, seul un filtre d'accumulation (16a) est traversé et une mesure de décalage est exécutée sur l'autre filtre à accumulation (16b).

2. Procédé selon la revendication 1, **caractérisé en ce que**
a) un intervalle comprend un long cycle de mesure et un cycle plus court d'étalonnage et/ou de rinçage et/ou
b) la cellule de mesure (7, 12) comporte plusieurs filtres à accumulation (16a, 16b) dont les cycles de mesure et de rinçage alternent et se complètent de telle sorte qu'un signal d'impédance de la cellule de mesure (7, 12) soit mesuré en permanence.

3. Procédé selon l'une des revendications 1 et 2, **caractérisé en ce que**
a) lors de l'étalonnage, la cellule de mesure (7, 12) est traversée par de l'eau propre et un signal d'étalonnage est formé,
b) lors du rinçage, la cellule de mesure (12) est traversée en sens inverse par de l'eau, en particulier par de l'eau contaminée par de l'huile, ou par un solvant et/ou
c) lors du rinçage, un filtre à accumulation (16, 16a, 16b) de la cellule de mesure (12) est nettoyé par ultrasons.

4. Procédé selon l'une des revendications 1 à 3, **caractérisé en ce que**
a) un signal de capacité est utilisé dans la cellule de mesure (7, 12) comme mesure de la concentration de l'huile dans l'eau,
b) la concentration en huile de l'eau qui traverse la cellule de mesure ou la concentration en huile accumulée dans un filtre à accumulation (16, 16a, 16b) de la cellule de mesure (7, 12) sont mesurées dans la cellule de mesure (7) et/ou
c) la concentration en huile dans l'eau est enrichie d'un facteur connu dans un filtre de séparation (2) raccordé en amont.
